# EUROPÄISCHE PATENTANMELDUNG

(11) **EP 0 994 092 A1**
(43) Veröffentlichungstag der Anmeldung: **19.04.2000**
(21) Anmeldenummer: 99118456.5
(22) Anmeldetag: 17.09.1999
(51) Int. Cl.: C07C 45/74, C07C 45/63, C07C 45/51, C07C 45/58, C07C 49/796, C07C 49/84, C07C 49/813, C07C 45/64, C07C 49/83, C07D 303/32

(54) **Verfahren zur Herstellung von substituierten Dibenzoylmethanverbindungen**

(30) Priorität: 16.10.1998 DE 19847778
(71) Anmelder: BASF AKTIENGESELLSCHAFT, 67056 Ludwigshafen (DE)
(72) Erfinder: Habeck, Thorsten, Dr., 67149 Meckenheim (DE); Prechtl, Frank, Dr., 60318 Frankfurt (DE)

(57) **Zusammenfassung**

Die Erfindung betrifft ein Verfahren zur Herstellung von substituierten Dibenzoylmethanverbindungen der allgemeinen Formel I, 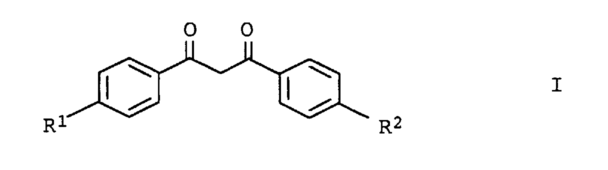 dadurch gekennzeichnet, daß man
a₁) Benzaldehyde der allgemeinen Formel II mit Acetophenonen der allgemeinen Formel III zu den Chalkonen der allgemeinen Formel IV kondensiert 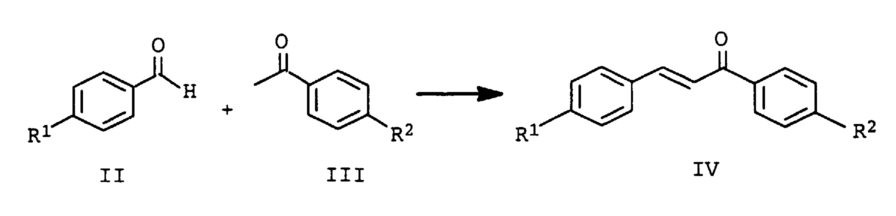 oder
a₂) Benzaldehyde der allgemeinen Formel V mit Acetophenonen der allgemeinen Formel VI zu den Chalkonen der allgemeinen Formel VII kondensiert und 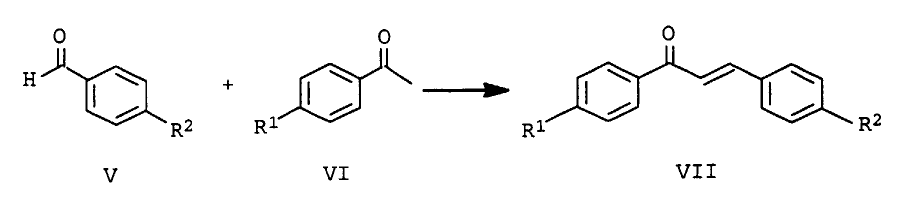
b) die Chalkone der Formeln IV und VII in die Dibenzoylmethanverbindungen der allgemeinen Formel I überführt, wobei die Substituenten R¹ und R² die in der Beschreibung genannten Bedeutungen haben.

## Beschreibung

Die Erfindung betrifft ein Verfahren zur Herstellung von substituierten Dibenzoylmethanverbindungen.

Sonnenlicht, das an die Erdoberfläche gelangt, hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Dermatologische Untersuchungen haben gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A- und UV-B-Bereich notwendig erscheinen.

Substanzen, die als Strukturelement eine Dibenzoylmethangruppe 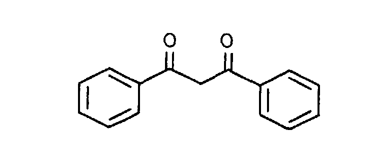 besitzen, zeichnen sich durch sehr gute Absorptionseigenschaften im UV-A Bereich aus. Aus dieser Stoffklasse gebräuchliche Lichtschutzfiltersubstanzen sind z.B. Eusolex® 8020 (nach INCI: Isopropyldibenzoylmethan, Fa. Merck) und Parsol® 1789 (nach INCI: Butylmethoxydibenzoylmethan, Fa. Givaudan).

DE-A-2945125 beschreibt ein Verfahren zur Herstellung von Parsol® 1789 durch Esterkondensation von 4-tert.-Butylbenzoesäuremethylester mit 4-Acetylanisol.

Aufgrund stetig steigender Nachfragen nach Lichtschutzmitteln, die als Strukturelement eine Dibenzoylmethangruppe besitzen, bestand die Aufgabe, ein Verfahren zur Herstellung substituierter Dibenzoylmethanverbindungen bereitzustellen, das einfach durchführbar ist und durch hohe Ausbeuten wirtschaftliche Vorteile erbringt.

Diese Aufgabe wurde gelöst durch ein Verfahren zur Herstellung substituierter Dibenzoylmethanverbindungen der allgemeinen Formel I, 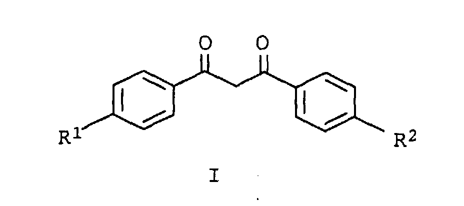 in der die Substituenten R¹ und R² unabhängig voneinander folgende Bedeutung haben:
- R¹: C₃-C₁₂-Alkyl;
- R²: Wasserstoff, C₃-C₁₂-Alkyl, C₁-C₁₂-Alkoxy,
dadurch gekennzeichnet, daß man
a₁) Benzaldehyde der allgemeinen Formel II mit Acetophenonen der allgemeinen Formel III zu den Chalkonen der allgemeinen Formel IV kondensiert, bei denen die exocyclische Doppelbindung in der E- oder Z-Konfiguration oder einer Mischung davon vorliegt und die Substituenten R¹ und R² die oben genannte Bedeutung haben 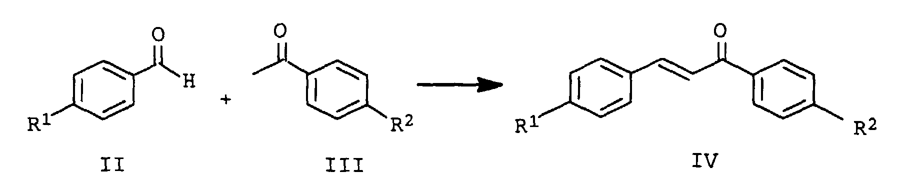 oder
a₂) Benzaldehyde der allgemeinen Formel V mit Acetophenonen der allgemeinen Formel VI zu den Chalkonen der allgemeinen Formel VII kondensiert, bei denen die exocyclische Doppelbindung in der E- oder Z-Konfiguration oder einer Mischung davon vorliegt und die Substituenten R¹ und R² die oben genannte Bedeutung haben und 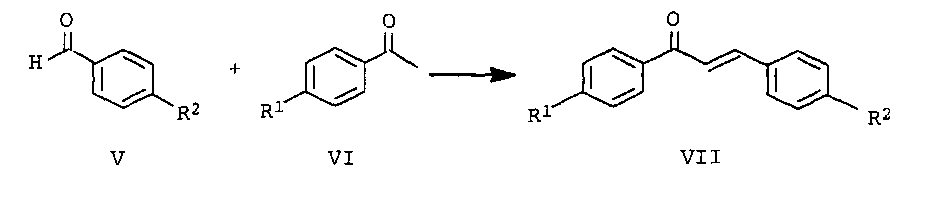
b) die Chalkone der Formeln IV oder VII in die Dibenzoylmethanverbindungen der allgemeinen Formel I überführt.

Als Alkylreste für R¹ und R² seien verzweigte oder unverzweigte C₃-C₁₂-Alkylketten, bevorzugt n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl genannt.

Besonders bevorzugte Alkylreste für R¹ und R² aus der oben genannten Gruppe sind die C₃-C₆-Alkylketten, ganz besonders bevorzugt die C₃-C₄-Alkylketten wie n-Propyl, 1-Methylethyl, n-Butyl, 1-Methylpropyl-, 2-Methylpropyl, 1,1-Dimethylethyl.

Als Alkoxyreste für R² kommen solche mit 1 bis 12 C-Atomen, vorzugsweise mit 1 bis 8 C-Atomen in Betracht.

Beispielsweise sind zu nennen:

| | |
|---|---|
| Methoxy- | Ethoxy- |
| iso-Propoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Besonders bevorzugte Alkoxyreste für R² sind solche mit 1 bis 6 C-Atomen, ganz besonders bevorzugt solche mit 1 bis 4 C-Atomen wie Methoxy, Ethoxy, iso-Propoxy, n-Propoxy, 1-Methylpropoxy, n-Butoxy.

Die Umsetzung der Benzaldehyde II bzw. V mit den Acetophenonderivaten III bzw. VI in den Verfahrensschritten a₁) bzw. a₂) erfolgt entsprechend den literaturbekannten Aldolkondensationen (siehe dazu: Organikum, VEB Deutscher Verlag der Wissenschaften, Berlin 1981, Seite 563-571; Indian J. Chem. Sec. B, 33, 1994, 455-459).

Die Kondensation kann sowohl basen- als auch säurekatalysiert erfolgen. Geeignete Katalysatoren sind:
- basische Alkali- und Erdalkalisalze, bevorzugt solche, die weder in den Edukten noch in den Produkten löslich sind und sich nach Reaktionsende leicht abtrennen lassen, besonders bevorzugt: Natrium-, Kalium- oder Calciumcarbonat oder Natriumhydrogencarbonat;
- Alkalihydroxide, bevorzugt Natrium- oder Kaliumhydroxid;
- Erdalkalioxide, bevorzugt Calcium- oder Magnesiumoxid;
- basische Zeolithe;
- Alkalialkoholate, beispielsweise Natriummethylat, Natriumethylat, Butyllithium;
- tertiäre Amine, wie z.B. Pyridin, Morpholin, Triethylamin, Triethanolamin;
- NH₃, NaNH₂, NH₄OAc;
- basisches Aluminiumoxid, basischer Ionenaustauscher;
- saure Katalysatoren, wie z.B. HCl, H₂SO₄, HNO₃, Phosphorsäure, Eisessig;
- saurer Ionenaustauscher, wie z.B. Lewatit® S100 (Fa. Bayer).

Die Menge der Katalysatoren beträgt im allgemeinen 1 bis 80 mol-%, bevorzugt 5 bis 50 mol-%, bezogen auf die Menge des eingesetzten Aldehyds.

Vorzugsweise arbeitet man bei Temperaturen von 10 bis 150°C, besonders 20 bis 100°C, besonders bevorzugt 25 bis 60°C. Besondere Bedingungen bezüglich des Druckes sind nicht erforderlich; im allgemeinen nimmt man die Umsetzung bei Atmosphärendruck vor.

Als Lösungsmittel können Alkohole, wie z.B. Methanol, Ethanol, propanol, Isopropanol, n-Butanol oder Isobutanol; Aromaten, wie z.B. Toluol oder Xylol; Kohlenwasserstoffe, beispielsweise Heptan oder Hexan; chlorierte Kohlenwasserstoffe, wie z.B. Chloroform oder Dichlormethan; Miglyol, Tetrahydrofuran eingesetzt werden. Die Reaktion kann aber auch ohne Lösungsmittel durchgeführt werden.

Die Reaktion kann sowohl diskontinuierlich als auch kontinuierlich durchgeführt werden. Bei der kontinuierlichen Fahrweise leitet man die Reaktionspartner vorzugsweise über ein Festbett aus einer unlöslichen Base, beispielsweise basische Zeolithe.

Die weitere Umsetzung der Chalkone IV oder VII zu den Dibenzoylmethanverbindungen der Formel I im Verfahrensschritt b) ist dadurch gekennzeichnet, daß man
b₁) die Chalkone IV oder VII durch Addition von Halogenen oder Hypohaliten in die Verbindungen der Formeln VIII oder IX überführt, 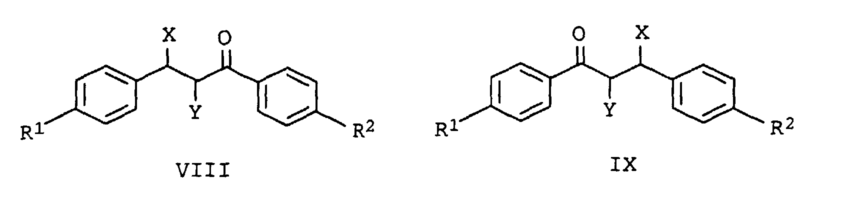 in der R¹ und R² die oben genannte Bedeutung haben und X für Halogen und OH steht und Y ein Halogen bedeutet und daß man
b₂) aus den Verbindungen VIII und IX durch Eliminierung von HY und gegebenenfalls anschließende Hydrolyse die Dibenzoylmethanverbindungen der allgemeinen Formel I herstellt.

Die für die Addition an die exocyclische Doppelbindung der Chalkone IV und VII verwendeten Halogene sind bevorzugt Brom oder Chlor, ganz besonders bevorzugt Chlor. Die Reaktion erfolgt in einfacher Weise durch Mischen der beiden Ausgangssubstanzen in einem inerten Lösungsmittel. Als Reaktionsprodukte entstehen Dihalogenverbindungen der Formeln VIII oder IX, in denen die Substituenten X und Y gemeinsam Brom oder Chlor, bevorzugt Chlor bedeuten.

Als Lösungsmittel kommen u.a. in Frage: aliphatische und aromatische Kohlenwasserstoffe wie Cyclohexan, Benzol, Toluol oder Xylol; halogenierte aliphatische und aromatische Kohlenwasserstoffe wie Tetrachlorkohlenstoff, Dichlormethan oder Chlorbenzol. Es ist aber auch möglich die Halogenaddition in Alkoholen wie beispielsweise Ethanol, Propanol durchzuführen.

Die Reaktionstemperaturen liegen im Bereich von -10°C bis 150°C, bevorzugt im Bereich von 0°C bis 80°C, besonders bevorzugt im Bereich von 5°C bis 50°C, ganz besonders bevorzugt im Bereich von 10°C bis 30°C.

Werden anstelle der Halogene Hypohalite wie z.B. Natrium Hypochlorit oder Natrium Hypobromit verwendet, so erhält man die entsprechenden Halogenhydrine der Formeln VIII oder IX, in denen X für OH steht und Y Halogen, bevorzugt Brom oder Chlor, besonders bevorzugt Chlor bedeutet.

Durch an sich bekannte, basenkatalysierte Eliminierung von HY, insbesondere HBr oder HCl, lassen sich die oben genannten Halogenhydrine in die gewünschten Dibenzoylmethanverbindungen der Formel I überführen.

Im Falle der Dihalogenide VIII oder IX (X=Y=Br oder Cl), erfolgt im Anschluß an die Eliminierung von HY eine basenkatalysierte Hydrolyse, beispielsweise in Gegenwart von Alkalialkoholaten wie Natriummethylat, bei Temperaturen zwischen 20 und 100°C, bevorzugt zwischen 30 und 95°C, besonders bevorzugt zwischen 40 und 90°C, die zu den gewünschten Dibenzoylmethanverbindungen der Formel I führt.

Eine weitere Möglichkeit um im Verfahrensschritt b) aus den Chalkonen IV und VII die Dibenzoylmethanverbindungen I herzustellen ist dadurch gekennzeichnet, daß man
b₁) die Chalkone IV oder VII durch Epoxidierung in die Oxirane der Formeln VIIIa oder IXa überführt, 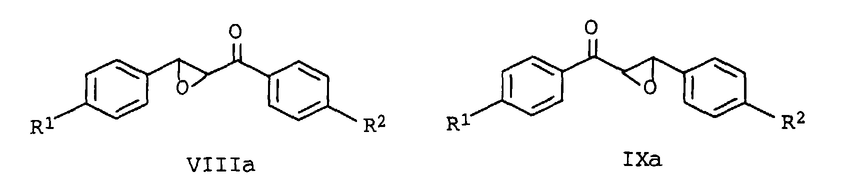 und man
b₂) aus den Epoxiden durch Ringöffnung die Dibenzoylmethanverbindungen der allgemeinen Formel I herstellt.

Die Epoxidierung der Chalkone IV und VII kann u.a. in an sich bekannten Weise in Gegenwart von Wasserstoffperoxid/NaOH im pH-Bereich von 8 bis 13, in Gegenwart von Peroxysäuren wie z.B. Peroxybenzoesäure oder m-Chlorperoxybenzoesäure, in Gegenwart von Dioxiranen wie z.B. Dimethyldioxiran, in Gegenwart von Hydroperoxiden wie z.B. tert. Butylhydroperoxid oder auch in Gegenwart von anorganischen Persulfaten wie z.B. Kaliumhydrogenpersulfat durchgeführt werden.

Die Reaktion erfolgt im allgemeinen in wäßrig-alkoholischen Lösungsmitteln bei Temperaturen von -20°C bis 150°C, bevorzugt von -10°C bis 80°C, besonders bevorzugt 0°C bis 60°C, ganz besonders bevorzugt von 0°C bis 30°C.

Die Öffnung des Oxiranrings kann in Gegenwart von Übergangsmetallkomplexen wie z.B. Tetrakis-triphenylphosphinpalladium erfolgen und wird u.a. beschrieben von Noyori et al., Angew. Chem. Int. Ed., 1984, 23, 847.

Das erfindungsgemäße Verfahren zeichnet sich dadurch aus, daß die damit hergestellten Dibenzoylmethanverbindungen der Formel I in hohen Ausbeuten und in technisch einfacher Weise zugänglich sind.

In einer besonderen Ausführungsform des erfindungsgemäßen Verfahrens werden entweder
a₁) Benzaldehyde der Formel II, bei denen R¹ für C₃-C₄-Alkyl steht, insbesondere 4-(1,1-Dimethylethyl)-benzaldehyd, mit Acetophenonderivaten der Formel III, bei denen R² für C₁-C₄-Alkoxy steht, insbesondere 4-Methoxyacetophenon, zu den entsprechenden Chalkonen der Formel IV, insbesondere zu 4-(1,1-Dimethylethyl)-benzyliden-4'-methoxy-acetophenon umgesetzt,
   oder
a₂) Benzaldehyde der Formel V, bei denen R² für C₁-C₄-Alkoxy steht, insbesondere 4-Methoxy-benzaldehyd, mit Acetophenonderivaten der Formel VI, bei denen R¹ für C₃-C₄-Alkyl steht, insbesondere 4-(1,1-Dimethylethyl)-acetophenon, zu den entsprechenden Chalkonen der Formel VII, insbesondere zu 4-Methoxy-benzyliden-4'-(1,1-dimethylethyl)-acetophenon umgesetzt,
   wobei die Chalkone IV und VII anschließend direkt und ohne vorherige Isolierung und Aufreinigung mittels Chloraddition, Eliminierung von HCl und anschließende basische Hydrolyse in die Dibenzoylmethanverbindungen der Formel I mit R¹ gleich C₃-C₄-Alkyl und R² gleich C₁-C₄-Alkoxy, insbesondere in 4-(1,1-Dimethylethyl)-4'-methoxydibenzoylmethan überführt werden.

Gegenstand der Erfindung sind ebenfalls Chalkone der allgemeinen Formel IV, 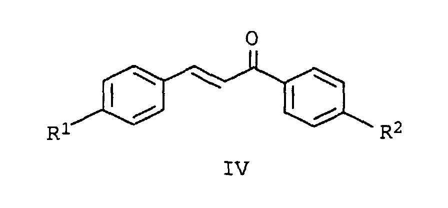 in der die exocyclische Doppelbindung in der E- oder Z-Konfiguration oder einer Mischung davon vorliegt und die Substituenten R¹ und R² unabhängig voneinander folgende Bedeutung haben:
- R¹: C₃-C₄-Alkyl;
- R²: Wasserstoff, C₁-C₄-Alkoxy.

Als Alkylreste für R¹ und R² seien verzweigte oder unverzweigte C₃-C₄-Alkylketten, bevorzugt n-Propyl, 1-Methylethyl, n-Butyl, 1-Methylpropyl-, 2-Methylpropyl, 1,1-Dimethylethyl genannt.

Als Alkoxyreste für R² kommen solche mit 1 bis 4 C-Atomen in Betracht.

Beispielsweise sind zu nennen:

| | |
|---|---|
| Methoxy- | Ethoxy- |
| iso-Propoxy- | n-Propoxy- |
| 1-Methylpropoxy- | n-Butoxy- |

Bevorzugt sind Chalkone der Formel IV, bei denen R¹ Isopropyl oder 1,1-Dimethylethyl bedeutet und R² für Wasserstoff oder Methoxy steht, insbesondere 4-(1,1-Dimethylethyl)-benzyliden-4'-methoxyacetophenon.

Gegenstand der Erfindung sind weiterhin Chalkone der allgemeinen Formel VII, 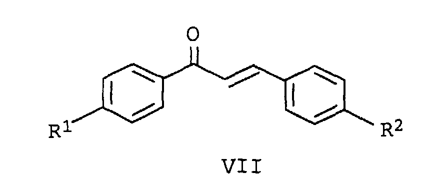 in der die exocyclische Doppelbindung in der E- oder Z-Konfiguration oder einer Mischung davon vorliegt und die Substituenten R¹ und R² unabhängig voneinander folgende Bedeutung haben:
- R¹: C₃-C₄-Alkyl;
- R²: Wasserstoff, C₁-C₄-Alkoxy.

Die genaueren Definitionen der Substituenten R¹ und R² für die Chalkone VII entsprechen denen von Verbindung IV.

Bevorzugt sind Chalkone der Formel VII, bei denen R¹ Isopropyl oder 1,1-Dimethylethyl bedeutet und R² für Wasserstoff oder Methoxy steht, insbesondere 4-Methoxy-benzyliden-4'-(1,1-Dimethylethyl)-acetophenon.

Gegenstand der Erfindung sind auch Diarylverbindungen der allgemeinen Formel VIII, 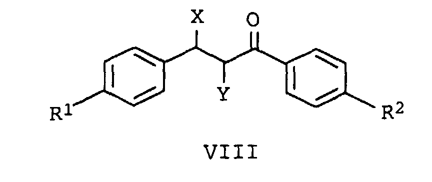 in der die Substituenten unabhängig voneinander folgende Bedeutung haben:
- X: Halogen, OH;
- Y: Halogen;
- R¹: C₃-C₁₂-Alkyl;
- R²: Wasserstoff, C₃-C₁₂-Alkyl, C₁-C₁₂-Alkoxy,
wobei die Substituenten X und Y zusammen mit den Kohlenstoffatomen, an die sie gebunden sind, auch einen Oxiranring bilden können.

Als Halogene für X und Y seien bevorzugt Brom und Chlor, besonders bevorzugt Chlor genannt.

Als Alkylreste für R¹ und R² seien verzweigte oder unverzweigte C₃-C₁₂-Alkylketten, bevorzugt n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl genannt.

Besonders bevorzugte Alkylreste für R¹ und R² aus der oben genannten Gruppe sind die C₃-C₆-Alkylketten, ganz besonders bevorzugt die C₃-C₄-Alkylketten wie n-Propyl, 1-Methylethyl, n-Butyl, 1-Methylpropyl-, 2-Methylpropyl, 1,1-Dimethylethyl.

Als Alkoxyreste für R² kommen solche mit 1 bis 12 C-Atomen, vorzugsweise mit 1 bis 8 C-Atomen in Betracht.

Beispielsweise sind zu nennen:

| | |
|---|---|
| Methoxy- | Ethoxy- |
| iso-Propoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Besonders bevorzugte Alkoxyreste für R² sind solche mit 1 bis 6 C-Atomen, ganz besonders bevorzugt solche mit 1 bis 4 C-Atomen wie Methoxy, Ethoxy, iso-Propoxy, n-Propoxy, 1-Methylpropoxy, n-Butoxy.

Bilden die Substituenten X und Y zusammen mit den Kohlenstoffatomen, an die sie gebunden sind, einen Oxiranring, so liegen Verbindungen der folgenden Struktur VIIIa vor.

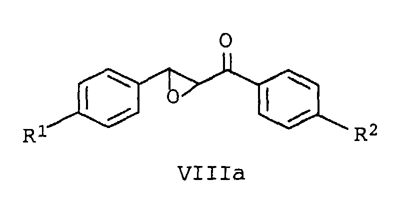

Bevorzugt sind Diarylverbindungen der Formel VIII, in der X und Y für OH, Br, Cl oder für einen, zusammen mit den Kohlenstoffatomen, an die sie gebunden sind, gebildeten Oxiranring stehen, R¹ C₃-C₆-Alkyl und R² Wasserstoff, C₃-C₆-Alkyl oder C₁-C₆-Alkoxy bedeuten.

Besonders bevorzugt sind Diarylverbindungen der Formel VIII, in der X und Y für Cl oder für einen, zusammen mit den Kohlenstoffatomen, an die sie gebunden sind, gebildeten Oxiranring stehen, R¹ C₃-C₆-Alkyl und R² Wasserstoff oder C₁-C₆-Alkoxy bedeuten.

Ganz besonders bevorzugt sind Diarylverbindungen der Formel VIII, in der X und Y für Cl oder für einen gemeinsamen, zusammen mit den Kohlenstoffatomen, an die sie gebunden sind, gebildeten Oxiranring stehen, R¹ C₃-C₄-Alkyl, insbesondere 1,1-Dimethylethyl bedeutet und R² für C₁-C₄-Alkoxy, insbesondere für Methoxy steht.

Gegenstand der Erfindung sind auch Diarylverbindungen der allgemeinen Formel IX, 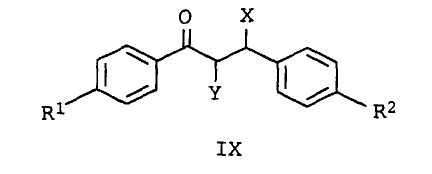 in der die Substituenten unabhängig voneinander folgende Bedeutung haben:
- X: Halogen, OH;
- Y: Halogen;
- R¹: C₃-C₁₂-Alkyl;
- R²: Wasserstoff, C₃-C₁₂-Alkyl, C₁-C₁₂-Alkoxy,
wobei die Substituenten X und Y zusammen mit den Kohlenstoffatomen, an die sie gebunden sind, auch einen Oxiranring, gemäß Formel IXa bilden können.

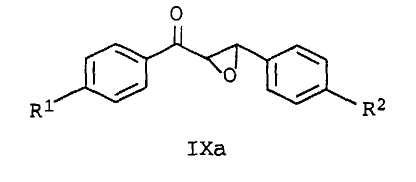

Die genaueren Definitionen der Substituenten R¹ und R² sowie X und Y von Verbindung IX entsprechen denen von Verbindung VIII.

Bevorzugt sind Diarylverbindungen der Formel IX, in der X und Y für OH, Br, Cl oder für einen, zusammen mit den Kohlenstoffatomen, an die sie gebunden sind, gebildeten Oxiranring stehen, R¹ C₃-C₆-Alkyl und R² Wasserstoff, C₃-C₆-Alkyl oder C₁-C₆-Alkoxy bedeuten.

Besonders bevorzugt sind Diarylverbindungen der Formel IX, in der X und Y für Cl oder für einen, zusammen mit den Kohlenstoffatomen, an die sie gebunden sind, gebildeten Oxiranring stehen, R¹ C₃-C₆-Alkyl und R² Wasserstoff oder C₁-C₆-Alkoxy bedeuten.

Ganz besonders bevorzugt sind Diarylverbindungen der Formel IX, in der X und Y für Cl oder für einen gemeinsamen, zusammen mit den Kohlenstoffatomen, an die sie gebunden sind, gebildeten Oxiranring stehen, R¹ C₃-C₄-Alkyl, insbesondere 1,1-Dimethylethyl bedeutet und R² für C₁-C₄-Alkoxy, insbesondere für Methoxy steht.

Die folgenden Beispiele sollen das erfindungsgemäße Verfahren näher erläutern.

### Beispiel 1

### Herstellung von 4-(1,1-Dimethylethyl)-benzyliden-4'-methoxyacetophenon

In einem 1 l Rundkolben mit Innenthermometer, Rückflußkühler und Blattrührer wurden 97,6 g (0,65 mol) 4-Methoxyacetophenon und 105,4 g (0,65 mol) 4-(1,1-Dimethylethyl)-benzaldehyd in 600 ml Methanol gelöst. Anschließend wurde bei Raumtemperatur mit 20 g (0,05 mol) 10 proz. NaOH versetzt. Danach wurde bei 30°C gerührt. Die gebildeten Kristalle wurden abfiltriert, mit 50 ml kaltem Methanol gewaschen und anschließend bei 75°C im Vakuum (150 mbar) getrocknet. Ausbeute: 172 g (90 % Ausbeute) schwach gelbe Kristalle; Schmp.: 114-116°C; Reinheit: > 99 %.

### Beispiel 2

### Herstellung von 4-(1,1-Dimethylethyl)-4'-methoxydibenzoylmethan

7,35 g (0, 025 mol) 4-(1,1-Dimethylethyl)-benzyliden-4'-methoxyacetophenon aus Beispiel 1 wurden in 50 ml Xylol suspendiert und bei 0 bis 5°C innerhalb von 1h mit 2 g (0,028 mol) Chlorgas umgesetzt. Zu dieser Lösung wurden 14,5 g (0,08 mol) einer 30 Gew.-prozentigen Natriummethylatlösung in Methanol gegeben und das Gemisch für 1h auf 80°C erwärmt. Anschließend versetzte man mit 8,0 g einer 37 Gew.-prozentigen wässrigen HCl-Lösung und rührte 2 h bei 80°C nach. Das ausgefallene NaCl wurde abfiltriert, das Lösungsmittel abdestilliert und der Rückstand aus Methanol umkristallisiert. Man erhielt 6,58 g farblose Kristalle mit einer Reinheit > 99 %. Die Ausbeute betrug 85 %.

### Beispiel 3

### Herstellung von 4'-Methoxyphenyl-[3-{4-(1,1-Dimethylethylphenyl)-oxiranyl}]-methanon

20,9 g (0,071 mol) 4-(1,1-Dimethylethyl)-benzyliden-4'-methoxyacetophen aus Beispiel 1 wurden in 350 ml EtOH suspendiert und bei 20 bis 25°C mit 11,5 ml einer 25 Gew.-prozentigen NaOH-Lösung versetzt. Hierauf tropfte man bei 20°C innerhalb von 10 min 9,65 g (0,085 mol) einer wäßrigen 30 Gew.-prozentigen Wasserstoffperoxid-Lösung und rührte 1h bei dieser Temperatur nach. Das Lösungsmittel wurde im Vakuum abdestilliert und der Rückstand durch Säulenchromatographie (SiO₂; Cyclohexan/Essigester) gereinigt. Man erhielt 17,6 g (80 % Ausbeute) eines gelblichen Öls.

### Beispiel 4

### Herstellung von 4-(1,1-Dimethylethyl)-4'-methoxydibenzoylmethan

1 g (3,2 mmol) des Epoxides aus Beispiel 3 wurden mit 40 mg (0,1 mmol) Tetrakis-triphenylphosphinpalladium und 0,1 g (0,3 mmol) Ethylen-1,2-diphenylphosphin versetzt und 7 h bei 140°C gerührt. Der Rückstand wurde durch Säulenchromatographie gereinigt. Man erhielt 0,94 g 4-(1,1-Dimethylethyl)-4'-methoxydibenzoylmethan (94 % Ausbeute) als farblose Kristalle mit einer Reinheit > 99 %.

## Patentansprüche

1. Verfahren zur Herstellung substituierter Dibenzoylmethanverbindungen der allgemeinen Formel I, 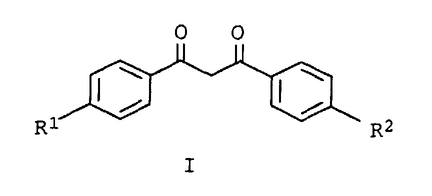 in der die Substituenten R¹ und R² unabhängig voneinander folgende Bedeutung haben:
R¹ C₃-C₁₂-Alkyl;
R² Wasserstoff, C₃-C₁₂-Alkyl, C₁-C₁₂-Alkoxy,
dadurch gekennzeichnet, daß man
a₁) Benzaldehyde der allgemeinen Formel II mit Acetophenonen der allgemeinen Formel III zu den Chalkonen der allgemeinen Formel IV kondensiert, bei denen die exocyclische Doppelbindung in der E- oder Z-Konfiguration oder einer Mischung davon vorliegt und die Substituenten R¹ und R² die oben genannte Bedeutung haben 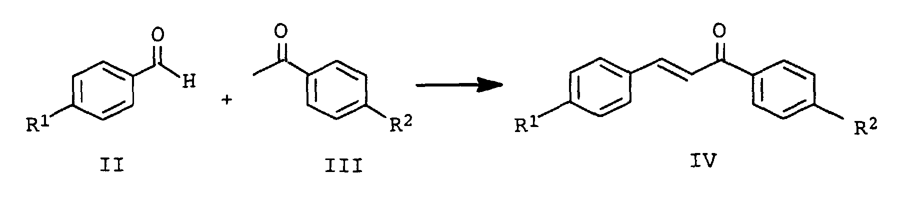 oder
a₂) Benzaldehyde der allgemeinen Formel V mit Acetophenonen der allgemeinen Formel VI zu den Chalkonen der allgemeinen Formel VII kondensiert, bei denen die exocyclische Doppelbindung in der E- oder Z-Konfiguration oder einer Mischung davon vorliegt und die Substituenten R¹ und R² die oben genannte Bedeutung haben und 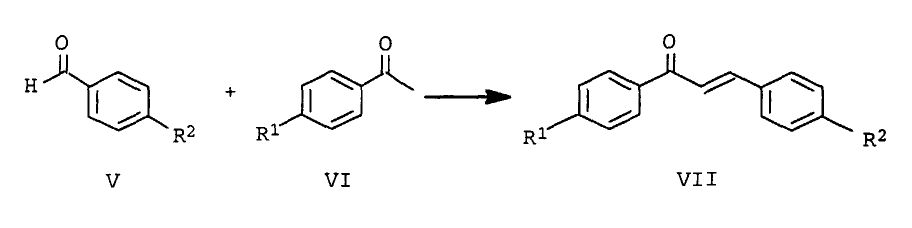
b) die Chalkone der Formeln IV oder VII in die Dibenzoylmethanverbindungen der allgemeinen Formel I überführt.

2. Verfahren nach Anspruch 1, dadurch gekennzeichnet, daß R¹ C₃-C₆-Alkyl und R² Wasserstoff, C₃-C₆-Alkyl oder C₁-C₆-Alkoxy bedeuten.

3. Verfahren nach einem der Ansprüche 1 oder 2, dadurch gekennzeichnet, daß R¹ C₃-C₆-Alkyl und R² Wasserstoff oder C₁-C₆-Alkoxy bedeuten.

4. Verfahren nach einem der Ansprüche 1 bis 3, dadurch gekennzeichnet, daß R¹ C₃-C₄-Alkyl und R² C₁-C₄-Alkoxy bedeuten.

5. Verfahren nach einem der Ansprüche 1 bis 4, dadurch gekennzeichnet, daß die Kondensation in den Verfahrensschritten a₁) und a₂) in Gegenwart einer Base erfolgt.

6. Verfahren nach einem der Ansprüche 1 bis 5, dadurch gekennzeichnet, daß man im Verfahrensschritt b) die Chalkone IV oder VII
b₁) durch Addition von Halogenen oder Hypohaliten in die Verbindungen der Formeln VIII oder IX überführt, 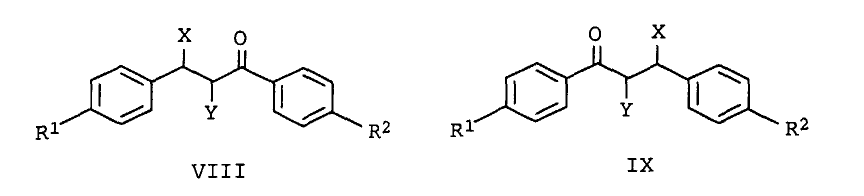 in der unabhängig voneinander X für Halogen oder OH steht und Y ein Halogen bedeuten und man
b₂) aus den Verbindungen VIII und IX durch Eliminierung von HY und gegebenenfalls anschließende Hydrolyse die Dibenzoylmethanverbindungen der allgemeinen Formel I herstellt.

7. Verfahren nach einem der Ansprüche 1 bis 5, dadurch gekennzeichnet, daß man im Verfahrensschritt b) die Chalkone IV oder VII
b₁) durch Epoxidierung in die Oxirane der Formeln VIIIa oder IXa überführt, 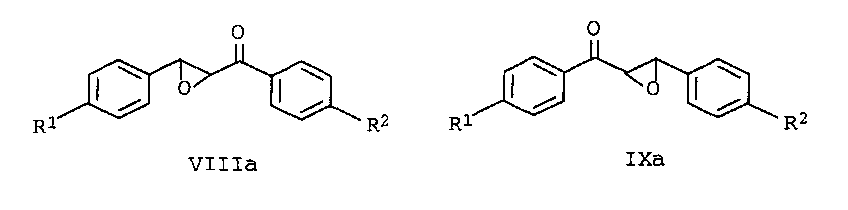 und man
b₂) aus den Oxiranen VIIIa und IXa durch Ringöffnung die Dibenzoylmethanverbindungen der allgemeinen Formel I herstellt.

8. Verfahren nach einem der Ansprüche 1 bis 7, dadurch gekennzeichnet, daß man die in den Verfahrensschritten a₁) und a₂) hergestellten Chalkone der Formeln IV und VII ohne Isolierung und Aufreinigung zu den Dibenzoylmethanverbindungen der allgemeinen Formel I umsetzt.

9. Chalkone der allgemeinen Formel IV, 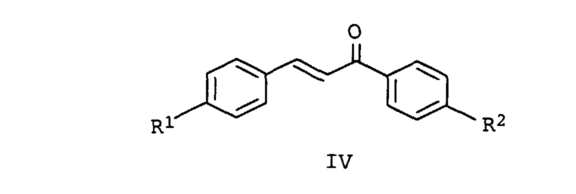 in der die exocyclische Doppelbindung in der E- oder Z-Konfiguration oder einer Mischung davon vorliegt und die Substituenten R¹ und R² unabhängig voneinander folgende Bedeutung haben:
R¹ C₃-C₄-Alkyl;
R² Wasserstoff, C₁-C₄-Alkoxy.

10. Chalkone nach Anspruch 9, dadurch gekennzeichnet, daß es sich um 4-(1,1-Dimethylethyl)-benzyliden-4'-methoxy-acetophenon handelt.

11. Chalkone der allgemeinen Formel VII, 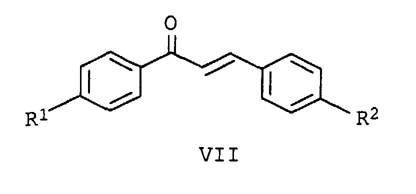 in der die exocyclische Doppelbindung in der E- oder Z-Konfiguration oder einer Mischung davon vorliegt und die Substituenten R¹ und R² unabhängig voneinander folgende Bedeutung haben:
R¹ C₃-C₄-Alkyl;
R² Wasserstoff, C₁-C₄-Alkoxy.

12. Chalkone nach Anspruch 11, dadurch gekennzeichnet, daß es sich um 4-Methoxy-benzyliden-4'-(1,1-dimethylethyl)-acetophenon handelt.

13. Diarylverbindungen der allgemeinen Formel VIII, 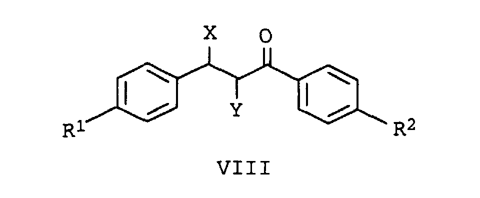 in der die Substituenten unabhängig voneinander folgende Bedeutung haben:
X Halogen, OH;
Y Halogen;
R¹ C₃-C₁₂-Alkyl;
R² Wasserstoff, C₃-C₁₂-Alkyl, C₁-C₁₂-Alkoxy,
wobei die Substituenten X und Y zusammen mit den Kohlenstoffatomen, an die sie gebunden sind, auch einen Oxiranring bilden können.

14. Diarylverbindungen nach Anspruch 13, in der X und Y für OH, Br, Cl oder für einen, zusammen mit den Kohlenstofffatomen, an die sie gebunden sind, gebildeten Oxiranring stehen, R¹ C₃-C₆-Alkyl und R² Wasserstoff, C₃-C₆-Alkyl oder C₁-C₆-Alkoxy bedeuten.

15. Diarylverbindungen nach einem der Ansprüche 13 oder 14, in der X und Y für Cl oder für einen, zusammen mit den Kohlenstoffatomen, an die sie gebunden sind, gebildeten Oxiranring stehen, R¹ C₃-C₆-Alkyl und R² Wasserstoff oder C₁-C₆-Alkoxy bedeuten.

16. Diarylverbindungen nach einem der Ansprüche 13 bis 15, in der X und Y für Cl oder für einen, zusammen mit den Kohlenstoffatomen, an die sie gebunden sind, gebildeten Oxiranring stehen, R¹ C₃-C₄-Alkyl bedeutet und R² für C₁-C₄-Alkoxy steht.

17. Diarylverbindungen nach einem der Ansprüche 13 bis 16, in der X und Y für Cl stehen, R¹ 1,1-Dimethylethyl und R² Methoxy bedeuten.

18. Diarylverbindungen nach einem der Ansprüche 13 bis 16, in der X und Y für einen, zusammen mit den Kohlenstoffatomen, an die sie gebunden sind, gebildeten Oxiranring stehen, R¹ 1,1-Dimethylethyl und R² Methoxy bedeuten.

19. Diarylverbindungen der allgemeinen Formel IX, 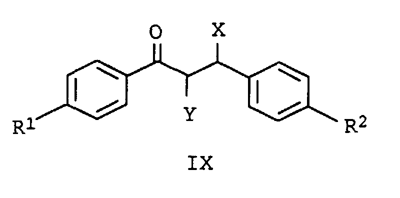 in der die Substituenten unabhängig voneinander folgende Bedeutung haben:
X Halogen, OH;
Y Halogen;
R¹ C₃-C₁₂-Alkyl;
R² Wasserstoff, C₃-C₁₂-Alkyl, C₁-C₁₂-Alkoxy,
wobei die Substituenten X und Y zusammen mit den Kohlenstoffatomen, an die sie gebunden sind, auch einen Oxiranring bilden können.

20. Diarylverbindungen nach Anspruch 19, in der X und Y für OH, Br, Cl oder für einen, zusammen mit den Kohlenstoffatomen, an die sie gebunden sind, gebildeten Oxiranring stehen, R¹ C₃-C₆-Alkyl und R² Wasserstoff, C₃-C₆-Alkyl oder C₁-C₆-Alkoxy bedeuten.

21. Diarylverbindungen nach einem der Ansprüche 19 oder 20, in der X und Y für Cl oder für einen, zusammen mit den Kohlenstoffatomen, an die sie gebunden sind, gebildeten Oxiranring stehen, R¹ C₃-C₆-Alkyl und R² Wasserstoff oder C₁-C₆-Alkoxy bedeuten.

22. Diarylverbindungen nach einem der Ansprüche 19 bis 21, in der X und Y für Cl oder für einen, zusammen mit den Kohlenstoffatomen, an die sie gebunden sind, gebildeten Oxiranring stehen, R¹ C₃-C₄-Alkyl bedeutet und R² für C₁-C₄-Alkoxy steht.

23. Diarylverbindungen nach einem der Ansprüche 19 bis 22, in der X und Y für Cl stehen, R¹ 1,1-Dimethylethyl und R² Methoxy bedeuten.

24. Diarylverbindungen nach einem der Ansprüche 19 bis 22, in der X und Y für einen, zusammen mit den Kohlenstoffatomen, an die sie gebunden sind, gebildeten Oxiranring stehen, R¹ 1,1-Dimethylethyl und R² Methoxy bedeuten.
